Europäisches Patentamt

European Patent Office

Office européen des brevets

(19)

(11) Publication number: **0 021 600**
**B1**

(12) **EUROPEAN PATENT SPECIFICATION**

(45) Date of publication of patent specification: **02.02.83**

(21) Application number: **80301698.9**

(22) Date of filing: **22.05.80**

(51) Int. Cl.³: **C 07 C  15/02,** C 07 C  2/66,
B 01 J  29/28

(54) Selective alkylation of xylenes with ethylene.

(30) Priority: **06.06.79 US  46394**

(43) Date of publication of application:
**07.01.81 Bulletin 81/1**

(45) Publication of the grant of the patent:
**02.02.83 Bulletin 83/5**

(84) Designated Contracting States:
**BE DE FR GB IT NL**

(56) References cited:
**GB - A - 1 536 828**
**US - A - 2 578 294**
**US - A - 4 016 218**

(73) Proprietor: **MOBIL OIL CORPORATION**
**150 East 42nd Street**
**New York New York 10017 (US)**

(72) Inventor: **Barile, George Conrad**
**7 Warner Drive**
**South Somerville, New Jersey (US)**
Inventor: **Kaeding, Warren William**
**700 Mountain Avenue**
**Westfield, New Jersey (US)**

(74) Representative: **Cooper, John Anthony**
**Mobil Court 3 Clements Inn**
**London WC2A 2EB (GB)**

Courier Press, Leamington Spa, England.

## Selective alkylation of xylenes with ethylene

This invention is concerned with a method for the selective preparation of 3,4-dimethyl-1-ethylbenzene from xylene and ethylene utilizing a shape selective zeolite catalyst.

Six structural isomers of the compound dimethylethylbenzene are known to exist. The names, structures, boiling points and equilibrium distribution at 315°C of these isomers are as shown below in Table I.

TABLE I

| Name and Structure | Boiling Point, °C | Equilibrium % at 315°C* |
|---|---|---|
| 2,3-Dimethyl-1-ethylbenzene | 193.9 | 3.2 |
| 2,4-Dimethyl-1-ethylbenzene | 188.4 | 16.4 |
| 2,5-Dimethyl-1-ethylbenzene | 186.9 | 23.9 |
| 2,6-Dimethyl-1-ethylbenzene | 190.0 | 1.9 |
| 3,4-Dimethyl-1-ethylbenzene | 189.8 | 20.9 |
| 3,5-Dimethyl-1-ethylbenzene | 183.8 | 33.7 |

*S. Csicsery, J. of Catalysis, *19*, 394 (1970)

A process for the synthetic manufacture of these compounds via ethylation of xylene or of mixed xylenes in the presence of aluminum chloride is disclosed in U.S. specification 3,855,331. However, the data disclosed therein demonstrates that even at low temperatures alkylation is not selective for any particular isomer of dimethylethylbenzene. Other literature on the transalkylation of 2-ethyltoluene on H-Mordenite [S. Csicsery, Journal of Catalysis, *23*, 124 (1971)] indicates that the zeolite is not particularly shape selective for the production of 3,4-dimethyl-1-ethylbenzene, yielding only 30% to 50% at between 200°C and 300°C.

U.S. Patent 4,016,218 describes the alkylation of aromatic hydrocarbons with $C_2$—$C_{20}$ olefins at 300 to 480°C, 100—21,000 kPa and WHSV 2—2000 over certain defined crystalline aluminosilicate zeolites. However, that patent is concerned almost exclusively with the monoalkylation of unsubstituted aromatic hydrocarbons and makes only the briefest possible mention of the alkylation of such alkylsubstituted aromatics as toluene and xylenes without suggesting that such a process would be selective for the production of specific polyalkylated aromatic hyrocarbons.

3,4-Dimethyl-1-ethylbenzene is an intermediate useful for the production of 3,4-dimethylstyrene. A process for the selective production of 3,4-dimethyl-1-ethylbenzene would eliminate the expense and waste of presently employed processes for isolating this isomer from equilibrium mixtures of the various dimethylethylbenzenes.

We have now discovered a process for the selective production of primarily a single desirable isomer of the six possible dimethylethylbenzene (i.e. 3,4-dimethyl-1-ethylbenzene). The process generally involves the alkylation of xylene and rearrangement of the various dimethylethyl-benzene isomers utilizing a particular type of crystalline zeolite catalyst thereby eliminating complex separation problems. Zeolite catalysts useful herein are characterized as having a silica to alumina ratio of at least about 12 and a Constraint Index as hereinafter defined, of greater than 2 and up to 12. The catalysts employed demonstrate a remarkable shape selectively to the 3,4-isomer among the various dimethylethylbenzene isomers, with the level of catalyst activity and selectivity being somewhat dependent on crystal size and steam modification.

The process may be carried out by contracting the reactants with the catalyst at a temperature of 250°C to 600°C and a pressure of $10^4$ N/m² to $10^7$ N/M² (0.1—100 atmospheres). The preferred temperatures and pressures will fall within the approximate range of 300°C to 450°C and $10^5$ to $4 \times 10^6$ N/m², respectively. The preferred catalyst for utilization herein comprises HZSM—5 crystalline zeolite. The significance and manner of determination of Constraint Index are described in our British Specification 1,446,522.

The class of zeolites defined herein is exemplified by ZSM—5, ZSM—11, ZSM—23 and ZSM—35, defined respectively by the X-ray data set forth in U.S. Specifications 3,702,886, 3,709,979, 4,076,842 and 4,016,245.

The specific zeolites described, when prepared in the presence of organic cations, are substantially catalytically inactive, possible because the intra-crystalline free space is occupied by organic cations from the forming solution. They may be activated by heating in an inert atmosphere at 540°C for one hour, for example, followed by base exchange with ammonium salts followed by calcination at 540°C in air. The presence of organic cations in the forming solution may not be absolutely essential to the formation of this type zeolite; however, the presence of these cations does appear to favor the formation of this special class of zeolite. More generally, it is desirable to activate this type of catalyst by base exchange with ammonium salts followed by calcination in air at about 540°C for from about 15 minutes to about 24 hours.

Natural zeolites may sometimes be converted to this type zeolite catalyst by various activation procedures and other treatments such as base exchange, steaming, alumina extraction and calcination, alone or in combinations. Natural minerals which may be so treated include ferrierite, brewsterite, stilbite, dachiardite, epistilbite, heulandite, and clinoptilolite.

In a preferred aspect of this invention, the zeolites hereof are selected as those having a crystal framework density, in the dry hydrogen form, of not less than about 1.6 grams per cubic centimetre. The dry density for known structures may be calculated from the number of silicon plus aluminium atoms per 1000 cubic Angstroms, as given, e.g., on Page 19 of the article on Zeolite Structure by W.M. Meier, included in "Proceedings of the Conference on Molecular Sieves, London, April 1967", published by the Society of Chemical Industry, London 1968. When the crystal structure is unknown, the crystal framework density may be determined by classical pyknometer techniques.

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable metal cations of Groups I to VIII of the Periodic Table, including, by way of example, nickel, copper, zinc, palladium, calcium or rare earth metals.

In practising the desired conversion process, it may be desirable to composite the above-described crystallising zeolite with another material resistant to the temperature and other conditions employed in the process. Such matrix material is useful as a binder and imparts greater resistance to the catalyst for the severe temperature, pressure and reactant feed stream velocity conditions encountered in many processes.

Useful matrix materials are disclosed in our European Specification 0006700. The zeolite suitably constitutes from 1 to 90 weight percent of a composite with such a binder.

The catalysts useful herein may desirably be modified by steaming. Such treatment entails contacting the zeolite with an atmosphere containing from 5% to 100% steam at a temperature of from 250°C to 1000°C for a period of between 0.25 and 100 hours and under pressures ranging from subatmospheric to several hundred atmospheres.

Another modifying treatment involves precoking of the catalyst to deposit a coating of between 2% and 75% by weight of coke thereon. Precoking can be accomplished by contacting the catalyst with a hydrocarbon charge, e.g. toluene, under high severity conditions or, alternatively, at a reduced hydrogen to hydrocarbon concentration (i.e. 0 to 1 mole ratio of hydrogen to hydrocarbon) for a sufficient time to deposit the desired amount of coke thereon.

It has been found that the level of activity and the degree of selectivity of the preferred catalyst is dependent to some degree on the crystal size of the catalyst. Catalysts useful in the present process include a range of crystal sizes from 0.01 to 40 microns. The smaller crystal sizes are the most preferred in that they have been found to give the most desirable level of selectivity to the 3,4-isomer. Although crystal sizes within the entire range as set out above have been found to be useful, the most preferred catalysts will be those having a crystal size within the range of 0.01 to 2 microns, more preferably still less than 1 micron.

Alkylation of xylene in the presence of the above-described catalyst is effected by contact of the xylene with ethylene at a temperature of between 250°C and 600°C, and preferably between 300°C and 450°C. The reaction generally takes place at atmospheric pressure, but the usable pressures fall within the range of $10^4$ N/m² to $10^7$ N/m². The molar ratio of xylene to ethylene will be most preferably within the range of 1:1 to 10:1. The reaction may be suitably accomplished utilizing a feed weight hourly space velocity (WHSV) of between 0.1 and 100, preferably between 1 and 20.

The process of this invention may be conducted with the organic reactants in either the gaseous or the liquid phase, or both. It may be carried out as a batch type, semi-continuous or continuous operation utilizing a fixed, fluidized, or moving bed catalyst system.

The following examples are presented by way of illustration of the process of the invention.

Example 1
Alkylation of isomeric dimethylbenzenes.
A catalyst comprising of 4.0g of HZSM—5 (65% by weight) on alumina was placed in a flow reactor and heated to 350°C. A feed system consisting of mixed dimethylbenzenes (22% ortho, 55%

3

meta, 23% para) and ethylene at a molar ratio of 3.5/l was passed across the catalyst at a weight hourly space velocity (WHSV) of 8.6/0.65 dimethylbenzene/ethylene and atmospheric pressure. The results are shown in Table II below.

TABLE II

| Catalyst | HZSM—5 | HZSM—5 |
|---|---|---|
| Time on Stream (hrs) | 0.5 | 2.0 |
| Liquid Effluent Analysis, wt%: | | |
| Benzene | 0.14 | 0.10 |
| Toluene | 1.05 | 0.94 |
| Dimethylbenzenes: | | |
| 1,2- | 19.74 | 19.52 |
| 1,3- | 46.40 | 45.56 |
| 1,4- | 18.90 | 18.38 |
| Ethyltoluenes | 1.68 | 1.67 |
| 1,3,5-Trimethylbenzene | 0.05 | 0.04 |
| 1,2,4-Trimethylbenzene | 0.40 | 0.32 |
| Dimethylethylbenzenes: | | |
| 2,3- | 0.19 | 0.21 |
| 2,4- | 1.01 | 1.23 |
| 2,5- | 0.71 | 0.84 |
| 3,4- | 6.52 | 8.09 |
| 3,5- | 0.51 | 0.51 |
| Other | 2.70 | 2.51 |
| Total | 100 | 100 |
| Selectivity, %: | | |
| 3,4-DMEB/total DMEB | 72.9 | 73.9 |
| 3,4-DMEB/total Products | 48.0 | 50.0 |

Example 2 (Comparison)

All conditions were identical to Example 1 except the catalyst was 4.0g of an amorphous silica-alumina cracking catalyst having 90/10 $SiO_2/Al_2O_3$. The results are shown in Table III below.

# 0 021 600

## TABLE III

| Catalyst | Amorphous Silica Alumina | Amorphous Silica Alumina |
|---|---|---|
| Time on Stream (hrs) | 0.5 | 1.0 |
| Liquid Effluent Analysis, wt%: | | |
| Benzene | 0.05 | — |
| Toluene | 0.50 | 0.44 |
| Dimethylbenzenes: 1,2- | 21.53 | 21.62 |
| 1,3- | 50.83 | 51.27 |
| 1,4- | 20.86 | 20.92 |
| Ethyltoluenes | 0.18 | 0.10 |
| 1,3,5-Trimethylbenzene | 0.19 | 0.17 |
| 1,2,4-Trimethylbenzene | 0.52 | 0.40 |
| Dimethylethylbenzenes: 2,3- | 0.16 | 0.17 |
| 2,4- | 0.95 | 0.90 |
| 2,5- | 0.94 | 0.87 |
| 3,4- | 1.38 | 1.24 |
| 3,5- | 1.42 | 1.25 |
| Other | 0.49 | 0.65 |
| Total | 100 | 100 |
| Selectivity, %: | | |
| 3,4-DMEB/total DMEB | 28.5 | 28.0 |
| 3,4-DMEB/total Products | 21.6 | 21.0 |

As will be seen from a comparison of Examples 1 and 2, both the total yield of 3,4-DMEB and the selectivity to 3,4-DMEB relative to other isomers of DMEB have been dramatically affected by the HZSM—5 catalyst. Selectivity to the 3,4-isomer in the ZSM—5 system of Example 1 has been enhanced 260% relative to the amorphous silica-alumina system (Example 2) run under identical conditions.

### Example 3 (Comparison)

The catalyst of Example 1 was replaced with 4.0g of H-Mordenite (Zeolon-200H) which had been calcined at 520°C for 8 hours prior to use. The operating parameters of the reaction were otherwise identical to those of the previous examples. The results are shown in Table IV below.

## TABLE IV

| Catalyst | H-Mordenite | H-Mordenite |
|---|---|---|
| Time on Stream (hrs) | 0.33 | 1.0 |
| **Liquid Effluent Analysis, wt%:** | | |
| Benzene | 0.09 | — |
| Toluene | 1.98 | 0.08 |
| **Dimethylbenzenes:** | | |
| 1,2- | 19.30 | 23.23 |
| 1,3- | 46.33 | 54.38 |
| 1,4- | 17.43 | 21.57 |
| Ethyltoluenes | 1.06 | — |
| 1,3,5-Trimethylbenzene | 0.29 | — |
| 1,2,4-Trimethylbenzene | 2.64 | — |
| **Dimethylethylbenzenes:** | | |
| 2,3- | 0.18 | — |
| 2,4- | 1.72 | 0.12 |
| 2,5- | 1.63 | 0.11 |
| 3,4- | 3.44 | 0.26 |
| 3,5- | 1.20 | — |
| Other | 2.71 | 0.25 |
| Total | 100 | 100 |
| **Selectivity, %:** | | |
| 3,4-DMEB/total DMEB | 42.1 | 53.1 |
| 3,4-DMEB/total Products | 22.1 | 45.6 |

As will be seen from the above results, the H-Mordenite catalyst aged quickly while on-stream and produced an isomeric mixture of dimethylethylbenzenes.

### Example 4 (Comparison)

Rare earth-exchanged Zeolite-Y (REY) was tested under conditions identical to those of Example 1. The catalyst had been calcined for 20 hours at 520°C prior to use. The results are summarized in Table V.

TABLE V

| Catalyst | REY | REY |
|---|---|---|
| Time on Stream (hrs) | 0.33 | 1.50 |
| Liquid Effluent Analysis, wt%: | | |
| Benzene | 0.23 | 0.09 |
| Toluene | 7.20 | 3.14 |
| Dimethylbenzenes: | | |
| 1,2- | 11.60 | 17.46 |
| 1,3- | 35.23 | 45.84 |
| 1,4- | 13.66 | 17.47 |
| Ethyltoluenes | 4.56 | 1.28 |
| 1,3,5-Trimethylbenzene | 2.79 | 0.84 |
| 1,2,4-Trimethylbenzene | 9.80 | 4.13 |
| Dimethylethylbenzenes: | | |
| 2,3- | 0.29 | 0.25 |
| 2,4- | 1.66 | 1.42 |
| 2,5- | 1.57 | 1.28 |
| 3,4- | 2.99 | 2.46 |
| 3,5- | 3.40 | 2.23 |
| Other | 5.02 | 2.11 |
| Total | 100 | 100 |
| Selectivity, %: | | |
| 3,4-DMEB/total DMEB | 30.2 | 32.2 |
| 3,4-DMEB/total Products | 7.7 | 12.7 |

Again, it will be seen that selectivity to the 3,4-DMEB is significantly less than that obtained with the HZSM—5 catalyst of Example 1.

Example 5 (Comparison)
Identical in all respects with Example 1 except in this instance the catalyst was 4.0g of HZSM—12 zeolite. The results are shown in Table VI below.

TABLE VI

| Catalyst | HZSM—12 | HZSM—12 |
|---|---|---|
| Time on Stream (hrs) | 0.50 | 1.0 |
| Liquid Effluent Analysis, wt%: | | |
| Benzene | 0.13 | 0.08 |
| Toluene | 4.40 | 2.11 |
| Dimethylbenzenes: | | |
| 1,2- | 18.02 | 20.48 |
| 1,3- | 42.40 | 48.74 |
| 1,4- | 16.59 | 18.73 |
| Ethyltoluenes | 1.54 | 1.62 |
| 1,3,5-Trimethylbenzene | 1.00 | 0.33 |
| 1,2,4-Trimethylbenzene | 3.75 | 1.75 |
| Dimethylethylbenzenes: | | |
| 2,3- | 0.22 | 0.13 |
| 2,4- | 1.64 | 1.18 |
| 2,5- | 1.62 | 1.18 |
| 3,4- | 2.97 | 2.10 |
| 3,5- | 2.66 | 1.21 |
| Other | 3.06 | 1.21 |
| Total | 100 | 100 |
| Selectivity, %: | | |
| 3,4-DMEB/total DMEB | 32.6 | 35.3 |
| 3,4-DMEB/total Products | 14.9 | 19.4 |

A side-by-side comparison of Table II (Example 1, HZSM—5) with Table VI above (HZSM—12) reveals an unexpected clear and substantial superiority of the ZSM—5 catalyst over the ZSM—12 catalyst with respect to selectivity to 3,4-DMEB as the product of choice in the ethylation of dimethylbenzenes.

Examples 6—12: Effect of Steaming the Catalyst.

The effects of steaming the catalyst for various time periods on the conversion and selectivities for alkylation were studied and the results are summarized in Table VII below. The steamed catalysts were prepared by passing a stream of water vapor over the catalyst at 4.3cm³/hr and 600°C. The water stream was shut off after steaming for the desired time and the temperature reduced to 550°C. The catalyst was then dried in flowing air at 550°C for 30 minutes. In each example the catalyst comprised 4.0g of HZSM—5 (65% on alumina) and the operating parameters were the same as in Example 1 — e.e. mixed dimethylbenzenes (22% ortho, 55% meta, 23% para) and ethylene at a 3.5/l molar ratio; WHSV 8.6/0.65; reaction temperature 350°C; atmospheric pressure.

8

## TABLE VII

### Effect of Steaming

| Example | Hours Steamed | 3,4-DMEB total DMEB | 3,4-DMEB total Products |
|---------|---------------|---------------------|-------------------------|
| 6 | None | 71.5% | 46.7% |
| 7 | 0.50 | 91.5 | 69.7 |
| 8 | 1.00 | 91.4 | 75.3 |
| 9 | 1.25 | 93.0 | 79.5 |
| 10 | 1.50 | 92.5 | 80.1 |
| 11 | 1.75 | 92.5 | 81.1 |
| 12 | 2.25 | 92.5 | 81.2 |

Steaming the catalyst prior to use is shown to dramatically increase the selectivity to 3,4-DMEB product. A similar effect may be obtained by depositing a coating of coke on the catalyst prior to use, or by a combination of precoking and steaming the catalyst.

### Examples 13—15: Alkylation of Individual Isomers of Dimethylbenzene

These examples demonstrate that the selectivity to 3,4-DMEB is independent of the dimethylbenzene isomer which is used as starting material. All of the run conditions were the same as in Example 1 with the exception that the dimethylbenzene feed was substantially the isomer as indicated in Table VIII below. The catalyst was 4.0g of HZSM—5 which had been steamed for 1.75 hours at 600°C prior to use and the DMEB/Ethylene feed WHSV was 8.6/0.65 at atmospheric pressure and 350°C.

## TABLE VIII

### Ethylation of Dimethylbenzene Isomers

| Example: | 13 | 14 | 15 |
|----------|------|------|------|
| Dimethylbenzene Isomer: | 1,2- | 1,3- | 1,4- |
| Liquid Products: | | | |
| Dimethylbenzenes: 1,2- | 65.46 | 6.00 | 2.79 |
| 1,3- | 15.44 | 69.10 | 17.89 |
| 1,4- | 4.44 | 7.40 | 65.63 |
| 3,4-DMEB | 10.43 | 13.22 | 10.28 |
| Total DMEB | 10.97 | 14.12 | 10.88 |
| Other | 3.69 | 3.38 | 2.8 |
| Selectivity, %: | | | |
| 3,4-DMEB/total DMEB | 95.1 | 93.6 | 94.5 |
| 3,4-DMEB/total Products | 71.1 | 75.5 | 75.1 |

9

The foregoing data clearly establish that the dimethylbenzene isomers do not come to equilibrium under the conditions of the reaction. What is more, the selectivity to 3,4-DMEB products is shown to be very high regardless of the isomeric structure of the dimethylbenzene starting material.

Examples 16—18: Effect of Catalyst Crystal Size

The effect of crystal size on the selectivities and conversion are illustrated by the following examples, wherein samples of HZSM—5 catalyst of three different crystal sizes were studied. The runs were carried out at 350°C and atmospheric pressure, the feed stream comprising ethylene and mixed dimethylbenzenes (22% ortho, 55% meta, 23% para). The reactant feed rates and nominal crystal sizes of the zeolite catalysts are given in Table IX below, as are the test results.

TABLE IX

Crystal Size Effect

| Example: | 16 | 17 | 18 |
|---|---|---|---|
| Catalyst: | HZSM—5 | HZSM—5 | HZSM—5 |
| Nominal Crystal Size (microns): | 0.02—0.05 | 0.2—0.5 | 1—2 |
| WHSV: | | | |
| Dimethylbenzene | 4.4 | 8.6 | 5.7 |
| Ethylene | 0.60 | 0.65 | 0.83 |
| Time on Stream (hrs): | 6 | 2 | 1 |
| Ethylene Conversion, mole % | 46.8 | 39 | 6.9 |
| Selectivity, %: | | | |
| 3,4-DMEB/total DMEB | 75.1 | 73.8 | 88.1 |
| 3,4-DMEB/total Products | 76.9 | 50.0 | 26.9 |

The foregoing illustrates that the overall selectivity to 3,4-DMEB among all products and the rate of conversion are relatively higher for the smaller crystal size zeolites. That is to say, with respect to HZSM—5, 0.02—0.5 >0.2—0.5 >1—2. However, the selectivity to 3,4-DMEB relative to all DMEB isomers produced is shown to increase with increasing crystal size.

**Claims**

1. A process for the selective alkylation of dimethylbenzene with ethylene to produce 3,4-dimethyl-1-ethylbenzene in excess of its normal equilibrium concentration, which comprises contacting dimethylbenzene with ethylene at a temperature of 250°C to 600°C and a pressure of $10^4$ N/m² to $10^7$ N/m² in the presence of a catalyst comprising a crystalline zeolite having a silica to alumina ratio of at least 12 and a constraint index of 2 to 12.

2. A process according to Claim 1, wherein alkylation is carried out at a temperature between 300°C and 450°C and a pressure of $10^5$ N/m² to $4 \times 10^6$ N/m².

3. A process according to Claim 1 or Claim 2, characterized in that zeolite is ZSM—5, ZSM—11, ZSM—23 or ZSM—35.

4. A process according to any preceding claim characterised in that the zeolite is in the form of crystals less than 1 micron in size.

10

**0 021 600**

5. A process according to any preceding claim characterized in that the zeolite is modified prior to use by steaming.

6. A process according to any preceding claim characterized in that the catalyst is modified prior to use by depositing between 2% and 75% by weight of coke thereon.

7. A process according to any preceding claim characterized in that the zeolite constitutes 1 to 90 weight percent of a composite with a binder.

**Revendications**

1. Procédé pour l'alcoylation sélective de diméthylbenzène par l'éthylène pour produire le 3,4-diméthyl-1-éthylbenzène en excès de sa concentration normale à l'équilibre, qui consiste à mettre en contact le diméthylbenzène avec l'éthylène à une température de 250°C à 600°C et une pression de $10^4$ N/m² à $10^7$ N/m² en présence d'un catalyseur constitué d'une zéolite cristalline présentant un rapport de silice à alumine d'au moins 12 et un indice de contrainte de 2 à 12.

2. Un procédé selon la revendication 1, dans lequel l'alcoylation est réalisée à une température entre 300°C et 450°C et une pression de $10^5$ N/m² à $4 \times 10^6$ N/m².

3. Un procédé selon la revendication 1 ou la revendication 2, caractérisé en ce que la zéolite est ZSM—5, ZSM—11, ZSM—23 ou ZSM—35.

4. Un procédé selon n'importe quelle revendication précédente, caractérisé en ce que la zéolite est sous la forme de cristaux de dimension inférieure à un micron.

5. Un procédé selon n'importe quelle revendication précédente, caractérisé en ce que la zéolite est modifiée avant l'usage par traitement à la vapeur.

6. Un procédé selon n'importe quelle revendication précédente, caractérisé en ce que le catalyseur est modifié avec l'usage par dépôt de 2 à 75% en poids de coke sur lui.

7. Un procédé selon n'importe quelle revendication précédente, caractérisé en ce que la zéolite constitue 1 à 90% en pourcentage pondéral d'une association avec un liant.

**Patentansprüche**

1. Verfahren zur selektiven Alkylierung von Dimethylbenzol mit Äthylen zur Herstellung von 3,4-Dimethyl-1-äthylbenzol im Überschuß von dessen normaler Gleichgewichtskonzentration, dadurch gekennzeichnet, daß man Dimethylbenzol mit Äthylen bei einer Temperatur von 250°C bis 600°C und einem Druck von $10^4$ N/m² bis $10^7$ N/m² in Gegenwart eines Katalysators in Berührung bringt, der einen kristallinen Zeolithen mit einem Siliciumdioxid/Aluminiumoxid-Verhältnis von wenigstens 12 und einem Zwangsindex von 2 bis 12 enthält.

2. Verfahren nach Anspruch 1, dadurch gekennzeichnet, daß die Alkylierung bei einer Temperatur zwischen 300°C und 450°C und einem Druck von $10^5$ N/m² bis $4 \times 10^6$ N/m² ausgeführt wird.

3. Verfahren nach Anspruch 1 oder 2, dadurch gekennzeichnet, daß der Zeolith ZSM—5, ZSM—11, ZSM—23 oder ZSM—35 ist.

4. Verfahren nach einem der vorhergehenden Ansprüche, dadurch gekennzeichnet, daß der Zeolith in Form von Kristallen mit einer Größe von weniger als 1 $\mu$m vorliegt.

5. Verfahren nach einem der vorhergehenden Ansprüche, daduch gekennzeichnet, daß der Zeolith vor Verwendung durch Dampfbehandlung modifiziert ist.

6. Verfahren nach einem der vorhergehenden Ansprüche, dadurch gekennzeichnet, daß der Katalysator vor Verwendung dadurch modifiziert worden ist, daß darauf zwischen 2 und 75 Gew.-% Koks niedergeschlagen worden sind.

7. Verfahren nach einem der vorhergehenden Ansprüche, dadurch gekennzeichnet, daß der Zeolith 1 bis 90 Gew.-% einer Mischung mit einem Bindemittel darstellt.